# EUROPEAN PATENT APPLICATION

(11) **EP 3 618 077 A1**
(43) Date of publication of application: **04.03.2020**
(21) Application number: 18190931.8
(22) Date of filing: 27.08.2018
(51) Int. Cl.: G16H 30/40

(54) **GENERATING METADATA FOR TRAINED MODEL**

(71) Applicant: Koninklijke Philips N.V., 5656 AE Eindhoven (NL)
(72) Inventor: BAKKER, Bart Jacob, 5656 AE Eindhoven (NL); MAVRIEUDUS, Dimitros, 5656 AE Eindhoven (NL); TRAJANOVSKI, Stojan, 5656 AE Eindhoven (NL)
(74) Representative: de Haan, Poul Erik

(57) **Abstract**

The invention relates to a trained model, such as a trained neural network, which is trained on training data. System and computer-implemented methods are provided for generating metadata which encodes a numerical characteristic of the training data of the trained model, and for using the metadata to determine conformance of input data of the trained model to the numerical characteristics of the training data. If the input data does not conform to the numerical characteristics, the use of the trained model on the input data may be considered out-of-specification ('out-of-spec'). Accordingly, a system applying the trained model to the input data may, for example, warn a user of the non-conformance, or may decline to apply the trained model to the input data, etc.

## Description

### FIELD OF THE INVENTION

The invention relates to a system and a computer-implemented method for processing a trained model, such as a trained neural network, which is trained on training data. The invention further relates to a system and a computer-implemented method for using the trained model with input data, for example, for classification of the input data. The invention further relates to a computer-readable medium comprising instructions to perform either computer-implemented method.

### BACKGROUND OF THE INVENTION

Machine learning is playing an increasingly important role in various domains and for various purposes. For example, in the medical domain, machine learning techniques such as deep learning have been found to be very suitable for classification and segmentation of image content from modalities including CT, X-ray, digital pathology and MRI. As is known per se, such machine learning may be used to train a model, such as a neural network, using training data as input. After such training, the trained model may be applied to new input data, e.g., to obtain a prediction from or classification of the new data. In a specific example, the trained model may be trained on labeled medical images and, during later use, applied to a medical image to obtain a classification or segmentation of an anatomical structure in the medical image. Various other uses of trained models are known.

A trained model is typically optimized and validated on a specific dataset. For example, in the example of medical image classification, a model may be trained on images and labels (or other classifications/annotations) from a particular modality. The trained model performance may be expected by users to be consistently accurate. However, this may require the input data, e.g., new images, to conform to the same or similar characteristics as the training data. In practice, it is not guaranteed that a trained model is used under specified conditions (also referred to 'in specification' or 'in-spec' use) as it may rather be used 'out-of-spec'.

For example, a user may apply a trained model to images which were acquired using a different image acquisition apparatus ('scanner') than was used to acquire the training images. Another example is that the settings of the scanner may change, image quality may degrade because of scratches on the lens or a defect in the scanning beam, the radiation dose may be different than required, etc.

### SUMMARY OF THE INVENTION

It may be desirable to facilitate in-spec use of a trained model.

The following aspects of the invention may involve generating metadata which encodes a numerical characteristic of the training data of a trained model, and using the metadata to determine conformance of input data of the trained model to the numerical characteristics of the training data. Accordingly, the system applying the trained model to the input data may, for example, warn a user that the use of the trained model with the input data is considered out of specification ('out-of-spec'), or may decline to apply the trained model to the input data, etc.

In accordance with a first aspect of the invention, a system is provided for processing a trained model. The system comprises:
a data interface for accessing:
   - model data representing a trained model, and
   - training data on which the trained model is trained;
a processor subsystem configured to:
   - characterize the training data by:
      applying the trained model to the training data to obtain intermediate output of the trained model, and
      determining the numerical characteristic based on the intermediate output of the trained model;
   - encode the numerical characteristic as metadata; and
   - associate the metadata with the model data to enable an entity applying the trained model to input data to determine whether the input data conforms to the numerical characteristic of the training data of the trained model.

In accordance with a further aspect of the invention, a computer-implemented method is provided for processing a trained model, comprising:
accessing:
   - model data representing a trained model, and
   - training data on which the trained model is trained;
characterizing the training data by:
   - applying the trained model to the training data to obtain intermediate output of the trained model, and
   - determining the numerical characteristic based on the intermediate output of the trained model;
encoding the numerical characteristic as metadata; and
associating the metadata with the model data to enable an entity applying the trained model to input data to determine whether the input data conforms to the numerical characteristic of the training data of the trained model.

In accordance with a further aspect of the invention, a system is provided for using a trained model. The system comprises:
a data interface for accessing:
   - model data representing a trained model having been trained on training data,
   - metadata associated with the model data and comprising a numerical characteristic, wherein the numerical characteristic is determined based on an intermediate output of the trained model when applied to the training data, and
   - input data to which the trained model is to be applied;
a processor subsystem configured to:
   - apply the trained model to the input data to obtain a further intermediate output of the trained model;
   - determine whether the input data conforms to the numerical characteristic of the training data of the trained model based on the further intermediate output; and
   - if the input data is determined not to conform to the numerical characteristic, generate an output signal indicative of said non-conformance.

In accordance with a further aspect of the invention, a computer-implemented method is provided for using a trained model, comprising:
accessing:
   - model data representing a trained model having been trained on training data,
   - metadata associated with the model data and comprising a numerical characteristic, wherein the numerical characteristic is determined based on an intermediate output of the trained model when applied to the training data, and
   - input data to which the trained model is to be applied;
applying the trained model to the input data to obtain a further intermediate output of the trained model;
determining whether the input data conforms to the numerical characteristic of the training data of the trained model based on the further intermediate output; and
if the input data is determined not to conform to the numerical characteristic, generating an output signal indicative of said non-conformance.

In accordance with a further aspect of the invention, a computer-readable medium is provided comprising transitory or non-transitory data representing instructions arranged to cause a processor system to perform either or both computer-implemented methods.

The above measures involve accessing a trained model, such as a trained neural network, and accessing training data on which the trained model is trained. The trained model may be applied to the training data in a manner as if the training data were input data to be processed by the trained model. For example, if the trained model is trained for image classification, the training data may be used as input to the trained model to perform image classification on the training data.

The above measures further involve determining a numerical characteristic from an intermediate output of the trained model. Here, the term 'intermediate output' is to be understood as follows: the trained model processes input data to generate output data, e.g., a classification, a probability, etc. To generate the output data, the trained model generates intermediate output data, being an internal precursor to the output data. As a non-limiting example, in the case of a trained neural network, such internal data may be activation values of hidden units of the trained neural network when applied to the input data. Other types of intermediate output are equally conceivable and dependent on the type of trained model. For example, if the trained model is a trained capsule network, the intermediate output may be one or more activity vectors of capsules. Similar examples exist for other models which are trainable by machine learning, e.g. latent variables in graphical models, such as Bayesian networks, etc.

The intermediate output may effectively represent an internal and intermediate characterization of the input data by the trained model, and when the input data is the training data, an intermediate characterization of the training data. This intermediate characterization may be made tangible by obtaining a numerical characteristic of the intermediate output. Thereby, a numerical characterization of the training data is obtained, albeit in an indirect rather than direct manner. Accordingly, the numerical characteristic is in the above and following also referred to as a numerical characteristic "of' the training data, even if it is not directly calculated from the training data but indirectly from the intermediate output.

The inventors have considered that such a numerical characteristic of the intermediate output of the trained model may be more suited for characterizing the training data than the training data itself. Namely, the training data may have properties which may make it less suitable for such characterization. For example, the training data may be large in terms of data size, which may make it computationally complex to calculate a descriptive numerical characteristic, and/or it may be difficult to manually (heuristically) or otherwise determine a concise characterization of the training data. The trained model, on the other hand, is typically trained to obtain such a concise characterization, e.g., a classification, probability, etc. However, while the output data itself (e.g., the classification or probability) is typically insufficiently descriptive across the range of possible input data, the intermediate output is well-suited to provide such a characterization.

The numerical characteristic may be encoded as metadata, and the metadata may be associated with the trained model, or more specifically with the model data of the trained model, in any suitable manner, e.g., by including the numerical characteristic in the model data itself, e.g., as a file header, XML element, etc., or providing the metadata as a separate file, or in any other manner.

At the application side, e.g., when using the trained model on non-training input data, the metadata may be used to determine whether current input data of the trained model conforms to the characteristic of the training data, and thus whether the use of the trained model with the current input data represents an 'in-spec' use or an 'out-of-spec' use. Namely, the intermediate output of the trained model may be compared or in another way validated against the numerical characteristic encoded in the metadata to determine conformance or non-conformance. If non-conformance is detected, this may be used to, e.g., warn the user, refrain from outputting the classification by the trained model, etc.

Optionally, the trained model is a trained neural network, and the intermediate output comprises activation values of a select subset of hidden units of the trained neural network. The activation values of hidden units may be well-suited for determining a numerical characteristic which is descriptive of the training data. A selection of such activation values may be used. In some examples, the trained neural network may be a trained deep neural network, e.g., having several layers of hidden units. The selection of activation values may correspond to a selection of select parts of select layers of the neural network.

Optionally, the training data comprises multiple training data objects, and the processor subsystem is configured to:
- apply the trained model to individual ones of the multiple training data objects to obtain multiple sets of activation values; and
- determine the numerical characteristic of the training data as a probability distribution of the multiple sets of activation values.

The training data may comprise multiple individual training data objects, e.g., multiple images, audio recordings, etc. In general, such training data objects may be characterized by a probability distribution. However, for many data types, obtaining a probability distribution directly from the training data itself is either infeasible or at least computationally complex, as the training data may be quite large (e.g., many high-resolution images), the probability distribution may be too complex to easily characterize, etc. Instead, a probability distribution of the activation values of a selection of hidden units may be generated, e.g., from the numerical outputs of selected parts of selected layers of a deep neural network. This may be less computationally complex as the number of hidden units may be significantly fewer than the number of data elements in each training data object.

Optionally, the processor subsystem is configured to:
- obtain out-of-spec data comprising multiple out-of-spec data objects which are different from the multiple training data objects;
- apply the trained neural network to individual ones of the multiple out-of-spec data objects to obtain further multiple sets of activation values;
- and select the subset of hidden units to establish a difference, or to increase or maximize the difference, between a) the probability distribution of the multiple sets of activation values and b) a probability distribution of the further multiple sets of activation values.

It may be desirable to select a specific subset of the hidden units which yields a distinct difference in probability distribution when calculating the probability distribution from the training data or from out-of-spec data. Such out-of-spec data may be considered, e.g., by manual assessment or by an automated metric, data which, when used as input to the trained model, represents an out-of-spec use of the trained model since the characteristics of the out-of-spec data do not conform to the characteristics of the training data, or at least not to a sufficient degree. In particular, the subset may be selected such that a sharp discrimination is obtained between what is considered to be in-spec data and out-of-spec data. For that purpose, out-of-spec data may be obtained which comprises multiple out-of-spec data objects which are different from the multiple training data objects. For example, in case of images, the out-of-spec data may comprise a set of images which have not been used in the training of the trained model and which are considered to represent out-of-spec input data for the trained model. The subset of hidden units, from which the probability distribution is obtained, may be selected to maximize the difference between the training data and the out-of-spec data, or at least to increase the difference compared to, e.g., a random selection or a selection of all hidden units.

For example, the processor subsystem may be configured to select the subset of hidden units by a combinatorial optimization method which optimizes the difference between a) the probability distribution of the multiple sets of activation values and b) the probability distribution of the further multiple sets of activation values, as a function of selected hidden units. The difference may, for example, be a Kullback-Leibler divergence measure, a cross entropy measure, or a mutual information measure, which are known per se.

Optionally, the processor subsystem is configured to:
- use a generator part of a generative adversarial network to generate negative samples on the basis of the training data;
- generate the out-of-spec data from the negative samples.

The generator part of a generative adversarial network (GAN) may be used to create the out-of-spec data in an automated manner, e.g., without manual selection or manual generation of the out-of-spec data, or in combination with a manual selection or manual generation of the out-of-spec data.

Optionally, the processor subsystem is configured to generate the model data by training a model using the training data, thereby obtaining the trained model. The system generating the metadata may in some embodiments be the same system as used for training the trained model. This may be convenient since the system already has access to the training data.

Optionally, the training data comprises multiple images, and the trained model is configured for image classification or image segmentation.

Optionally, the system for using the trained model further comprises an output interface for outputting the output signal to a rendering device for rendering the output signal in a sensory perceptible manner to a user. For example, the system may generate a warning message on a display.

Optionally, the trained model is a trained neural network, the numerical characteristic is a probability distribution obtained from multiple sets of activation values of a subset of hidden units of the trained neural network, the multiple sets of activation values are obtained by applying the trained model to the training data, the further intermediate output of the trained model comprises a further set of activation values of the subset of hidden units, and the processor subsystem of the system for using the trained model is configured to:
- determine a probability of the further set of activation values based on the probability distribution; and
- determine whether the input data conforms to the numerical characteristic of the training data of the trained model as a function of the probability.

The above may represent a specific example of how the system applying the trained model may determine whether the input data conforms to the numerical characteristic of the training data of the trained model.

It will be appreciated by those skilled in the art that two or more of the above-mentioned embodiments, implementations, and/or optional aspects of the invention may be combined in any way deemed useful.

Modifications and variations of any computer-implemented method and/or any computer program product, which correspond to the described modifications and variations of a corresponding system, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system for processing a trained model to generate metadata for the trained model, the metadata encoding a numerical characteristic of the training data on which the trained model is trained;
Fig. 2 shows a system for using the trained model with input data and for using the metadata to determine whether the input data conforms to the numerical characteristic of the training data of the trained model;
Fig. 3 shows a detailed example of how a numerical characteristic may be determined for a trained neural network, being in this example a probability function of activation values of a select subset of hidden units of said network;
Fig. 4 shows a method of processing a trained model to generate metadata which encodes a numerical characteristic of the model's training data;
Fig. 5 shows a method of using the trained model with input data and for using the metadata to determine whether the input data conforms to the numerical characteristic of the training data of the trained model; and
Fig. 6 shows a computer-readable medium comprising data.

It should be noted that the figures are purely diagrammatic and not drawn to scale. In the figures, elements which correspond to elements already described may have the same reference numerals.

### List of abbreviations

- DL: Deep Learning
- DNN: Deep Neural Network
- GAN: Generative Adversarial Network
- GenAl: Genetic Algorithm
- KL: Kullback-Leibler

### List of reference numbers

The following list of reference numbers is provided for facilitating the interpretation of the drawings and shall not be construed as limiting the claims.
- 020, 022: data storage
- 030: training data
- 040: input data
- 050: model data
- 060: metadata

- 100: system for processing trained model
- 120: data interface
- 122, 124: data communication
- 140: processor subsystem
- 200: system for using trained model
- 220: data interface
- 222, 224: data communication
- 240: processor subsystem
- 242: output signal
- 260: display output interface
- 262: display data
- 280: display
- 282: warning dialog box

- 300: training data
- 310: generative adversarial network
- 320: trained model
- 330: probability distribution (in-spec)
- 335: probability distribution (out-of-spec)
- 340: difference measure (Kullback-Leibler divergence)
- 350: combinatorial optimization method (genetic algorithm)
- 360: activation values of subset of hidden units, variance

- 400: method of processing trained model
- 410: accessing model data, training data
- 420: characterizing training data
- 430: applying trained model to training data
- 440: determining numerical characteristic
- 450: encoding numerical characteristic as metadata
- 460: associating metadata with model data

- 500: method of using trained model
- 510: accessing model data, metadata, input data
- 520: applying trained model to input data
- 530: determining conformance of input data
- 540: generating output signal indicative of non-conformance
- 600: computer-readable medium
- 610: non-transitory data

### DETAILED DESCRIPTION OF EMBODIMENTS

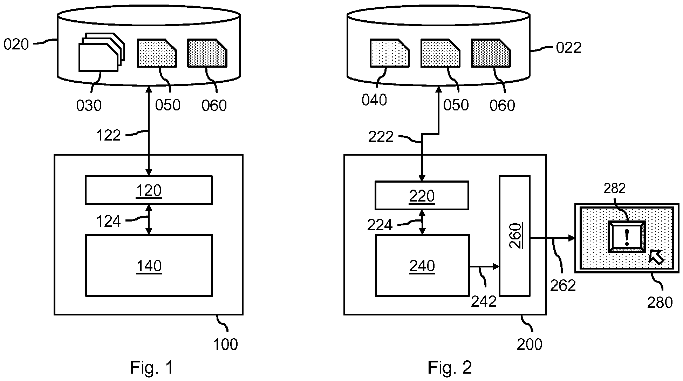

**Fig. 1** shows a system 100 for processing a trained model to generate metadata for the trained model which encodes a numerical characteristic of the training data on which the trained model is trained. The system 100 may comprise a data interface 120 and a processor subsystem 140 which may internally communicate via data communication 124. The processor subsystem 140 may be configured to, during operation of the system 100 and using the data interface 120, access model data 050 representing a trained model, and access training data 030 on which the trained model is trained. For example, as shown in Fig. 1, the data interface 120 may provide access 122 to an external data storage 020 which may comprise said data 030, 050. Alternatively, the data 030, 050 may be accessed from an internal data storage which is part of the system 100. Alternatively, the data 030, 050 may be received via a network from another entity. In general, the data interface 120 may take various forms, such as a network interface to a local or wide area network, e.g., the Internet, a storage interface to an internal or external data storage, etc. The data storage 020 may take any known and suitable form.

The processor subsystem 140 may be further configured to, during operation of the system 100, characterize the training data by applying the trained model to the training data to obtain intermediate output of the trained model, and determining a numerical characteristic based on the intermediate output of the trained model. The processor subsystem 140 may encode the numerical characteristic as metadata 060, and associate the metadata 060 with the model data 050 to enable an entity applying the trained model to input data to determine whether the input data conforms to the numerical characteristic of the training data of the trained model. An example of such an entity is the system 200 of Fig. 2.

The metadata 060 may be stored by the system 100 in the data storage 020 or elsewhere, sent via a network, etc. In general, the metadata 060 may be stored in a same data container as the training data 050, for example in a same file(s), but may also be provided as separate metadata 060 which is associated with the training data 050. For example, in some embodiments, the training data 050 may link to the metadata 060, e.g., by containing an URL at which the metadata 060 is accessible, or the metadata 060 may link to the training data 050. Various other means of association are equally conceivable and within reach of the skilled person.

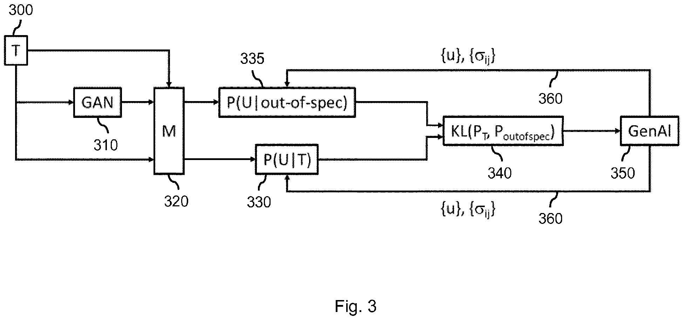

Various details and aspects of the operation of the system 100 will be further elucidated with reference to Fig. 3, including optional aspects thereof.

In general, the system 100 may be embodied as, or in, a single device or apparatus, such as a workstation, e.g., laptop or desktop-based, or a server. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the data interface and the processor subsystem, may be implemented in the device or apparatus in the form of programmable logic, e.g., as a Field-Programmable Gate Array (FPGA). In general, each functional unit of the system may be implemented in the form of a circuit. It is noted that the system 100 may also be implemented in a distributed manner, e.g., involving different devices or apparatuses, such as distributed servers, e.g., in the form of cloud computing.

**Fig. 2** shows a system 200 for using the trained model with input data and for using the metadata to determine whether the input data conforms to the numerical characteristic of the training data of the trained model. The system 200 may comprise a data interface 220 and a processor subsystem 240 which may internally communicate via data communication 224. The processor subsystem 240 may be configured to, during operation of the system 200 and using the data interface 220, access the model data 050 and the metadata 060 as described with reference to Fig. 1, as well as input data 040 to which the trained model is to be applied. For example, as also shown in Fig. 2, the data interface 220 may provide access 222 to an external data storage 022 which comprises said data 040-060. Alternatively, the data 040-060 may be accessed from an internal data storage. Alternatively, the data 040-060 may be received via a network. In general, the data interface 220 may take various forms, such as a network interface to a local or wide area network, e.g., the Internet, a storage interface to an internal or external data storage, etc. The data storage 022 may take any known and suitable form.

The processor subsystem 240 may be further configured to, during operation of the system 200, apply the trained model 050 to the input data 040 to obtain a further intermediate output of the trained model, determine whether the input data 040 conforms to the numerical characteristic of the training data of the trained model based on the further intermediate output, and if the input data is determined not to conform to the numerical characteristic, generate an output signal 242 indicative of said non-conformance. As an optional component, the system 200 may comprise a display output interface 260 or any other type of output interface for outputting the output signal 242 to a rendering device, such as a display 280. For example, the display output interface 260 may generate display data 262 for the display 280 which causes the display 280 to render the output signal in a sensory perceptible manner, e.g., as an on-screen warning dialog box 282.

In general, the system 200 may be embodied as, or in, a single device or apparatus, such as a workstation, e.g., laptop or desktop-based, or a mobile device. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the data interface and the processor subsystem, may be implemented in the device or apparatus in the form of programmable logic, e.g., as a Field-Programmable Gate Array (FPGA). In general, each functional unit of the system may be implemented in the form of a circuit. It is noted that the system 200 may also be implemented in a distributed manner, e.g., involving different devices or apparatuses, such as the client and server of a client-server implementation.

**Fig. 3** shows a detailed yet non-limiting example of how a numerical characteristic may be determined for a trained model, for example, by the system 100 of Fig. 1. In this example, the trained model is a trained neural network, and more specifically a Deep Neural Network (DNN) model trained by deep learning (DL). For that purpose, deep learning techniques may be used, as known per se, which may be based on error function minimization, gradient descent methods, error back-propagation and (specifically in deep learning) mini-batch optimization, etc. The aforementioned techniques may be considered textbook methods, but other suitable techniques may be used as well. This trained model 320 may take the form of a deep neural network **M** with a suitable network architecture and a set of hidden units ***u*.** Fig. 3 further shows training data **T** which may be used for several purposes. Firstly, the training data **T** may be used to train the deep learning model **M.** Secondly, the training data **T** may be used to estimate a Generative Adversarial Network (**GAN**) 310 (Goodfellow, 2014, see reference section) which maybe used to generate so-termed 'out-of-spec' samples which are close to the distribution of **T,** but do not actually derive from this distribution, e.g., are not actual observations like the training data **T.** Alternatively, the out-of-spec data may be obtained from another source, e.g., from actual observations, e.g., from acquired images which are considered to represent out-of-spec images. The trained model **M** may be applied to both the out-of-spec data and to the training data **T.** This may result in the generation of model outputs, including all intermediate model outputs **U.**

Two probability distributions, namely **P(U|out-of-spec)** 335 and **P(U|T)** 330 may describe the sampling probability of selected intermediate model unit outputs **{u}.** Such selected intermediate model units may for example correspond to selected hidden units of the trained model **M,** with their outputs corresponding to their activation values. Such hidden units may also be simply referred to as units and their activation values as unit activations or simply as intermediate model output. A normal distribution of the activation values of these units **{u}** may be characterized in terms of means **µᵢ** and covariance **σᵢⱼ**, where the indexes **i** and **j** correspond to all units in **{u}** that have been selected. Note that **{u}** may be a subset of the complete set of hidden units of the model **M.** In some cases, a large number of units in **{u}** may necessitate a simple distribution, where **σᵢⱼ** = 0 if **i** ≠ **j**. However, in general, σᵢⱼ may be selected with or without off-diagonal non-zero values. The selection of units in the distribution and which σᵢⱼ are non-zero may be represented in Fig. 3 as **{u},** {**σᵢⱼ**} 360.

Given selection **{u},** {**σᵢⱼ**}, the training data **T** and the (GAN-generated) out-of-spec data, the distributions **P(U|out-of-spec)** and **P(U|T)** may be estimated, e.g., by simply taking means and (co-)variances of the unit activations that result from applying the trained model M to the data objects of the training data T and the out-of-spec data, respectively. A selection of **{u},** {**σᵢⱼ**} may be preferred where the difference between the two distributions is maximal, as a probability distribution is desired that can distinguish samples that come from the training data **T,** or from an identical source as the training data, from the aforementioned type and other types of out-of-spec data. This difference may be expressed by the Kullback-Leibler distance 340, cross-entropy, mutual information or other measures as known per se.

To maximize the difference between the distributions **P(U|out-of-spec)** and **P(U|T)**, a combinatorial optimization method 350 may be used, e.g., from the family of genetic algorithms ('GenAl'), integer linear programming, etc. Alternatively, other combinatorial optimization methods as known per se in the field may be used.

As a result, a **P(U|T)** and **P(U|out-of-spec)** may be obtained which may be optimal, or at least well-suited, to distinguish samples from the training data **T** from out-of-spec data samples. One may consider to use **P(U|out-of-spec)** to estimate whether a new input data object is to be considered out-of-spec. However, it may be preferable to use **P(U|T)** as an in-spec detector instead, as one may aim for the training data **T** to be a complete description of in-spec data, whereas for the out-of-spec data, it may be difficult, if not impossible, to determine whether all out-of-spec situations have been covered. As such, **P(U|T)** may be encoded as metadata to the deep learning model **M.** When using the deep learning model **M** on new input data, one may estimate whether an input data object, such as an image, is to be considered in-spec or out-of-spec by using the input data object as input to the deep learning model **M** to estimate **U_{new}** and by assigning an in-spec probability **P(U_{new} |T)** to the input data object, using the distribution function **P(U|T)** provided by the metadata. For example, if the in-spec probability **P(U_{new} |T)** exceeds a pre-set threshold θ, the input data object may be considered in-spec and else out-of-spec.

It is noted that when the trained model is applied to images as training data, as out-of-spec data or as 'new' input data, each image may, when input to the trained model, result in a vector in which each element *I* may correspond to the activation value of hidden unit i. This vector may be seen as one sample drawn from an n-dimensional distribution (n being the number of hidden units considered / the length of the vector), which may for example be described by a mean **µ** (of length n) and variance matrix **σ** (n x n). During training, multiple of such images may define the distribution, e.g., by mean **µ** and variance **σ**, which may be calculated directly from the collection of activation vectors. The above may also apply to other training data which comprises multiple training data objects, mutatis mutandis.

With further reference to the out-of-spec data: instead or in addition to this data being generated by a GAN 310, the data may be acquired from elsewhere, e.g., in a same or similar manner as the training data **T** is acquired, e.g., from a medical scan apparatus. However, while the training data **T** may be annotated in order to perform supervised training of the model **M,** the out-of-spec data may not need to be annotated as the model **M** is not trained based on this data. For example, if the trained model is used for medical image classification, the out-of-spec data may be obtained by filtering logging information of medical scan apparatuses for known out-of-spec usages, and by using the image(s) acquired by such out-of-spec usages as the out-of-spec data. For example, machine operation logs of a CT scanner or a pathology image scanner, and possible logged protocols, e.g., denoting the preparation of a pathology tissue sample, may be filtered. Such out-of-spec data may also be gathered in similar ways in non-image based medical contexts, e.g., in the case of EEG or ECG data, or in general in non-medical contexts. Thereby, out-of-spec data objects may be generated that contribute to obtaining a **P(U|T)** that describes the in-spec usage more precisely, e.g., to draw sharp borders between in- and out-of-spec data. With continued reference to **Fig. 3****,** the out-of-spec data may be used as input to the deep learning model **M** at the location/instead of the **GAN.**

In general, the **GAN** 310 may be used to generate out-of-spec data in a more general manner, as it does not rely on having to acquire out-of-spec data. Using the GAN generator directly may not be optimal, as the generator may be of such high quality that a human may have difficulty distinguishing generated from real data. However, it is known to apply a GAN to produce so-called 'negative samples', which may be used as out-of-spec samples in the present context. For example, this is known from Yu 2017, Dai 2017, Wang

2018, and Zheng 2018, which are hereby incorporated by reference in as far as describing the generating of such negative samples. The generating of such negative samples may involve a trade-off between being outside of the 'in-spec' characteristics, e.g., the characteristics of the training data T, but close enough to these characteristics so as to allow a sharp boundary to be defined between in- and out-of-spec data. Finally, a mixture of GAN-generated out-of-spec data and otherwise acquired out-of-spec data may be used as well.

Although the above describes the numerical characteristic to be a probability distribution, various other types of numerical characterizations may be used as well, as known per se from field of statistics. Moreover, if a probability distribution is used as numerical characterization, any known and suitable type, presentation, or way of calculating the probability distribution may be used.

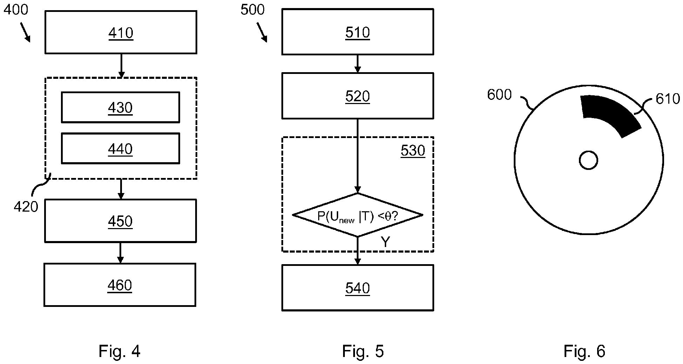

**Fig. 4** shows a block-diagram of computer-implemented method 400 for processing a trained model. The method 400 may correspond to an operation of the system 100 of Fig. 1. However, this is not a limitation, in that the method 400 may also be performed using another system, apparatus or device.

The method 400 may comprise, in an operation titled "ACCESSING MODEL DATA, TRAINING DATA", accessing 410 model data representing a trained model, and training data on which the trained model is trained. The method 400 may further comprise, in an operation titled "CHARACTERIZING TRAINING DATA", characterizing 420 the training data by, in an operation titled "APPLYING TRAINED MODEL TO TRAINING DATA", applying 430 the trained model to the training data to obtain intermediate output of the trained model, and in an operation titled "DETERMINING NUMERICAL CHARACTERISTIC", determining 440 the numerical characteristic based on the intermediate output of the trained model. The method 400 may further comprise, in an operation titled "ENCODING NUMERICAL CHARACTERISTIC AS METADATA", encoding 450 the numerical characteristic as metadata, and in an operation titled "ASSOCIATING METADATA WITH MODEL DATA", associating 460 the metadata with the model data to enable an entity applying the trained model to input data to determine whether the input data conforms to the numerical characteristic of the training data of the trained model.

**Fig. 5** shows a block-diagram of computer-implemented method 500 for using a trained model. The method 500 may correspond to an operation of the system 200 of Fig. 2. However, this is not a limitation, in that the method 500 may also be performed using another system, apparatus or device.

The method 500 may comprise, in an operation titled "ACCESSING MODEL DATA, METADATA, INPUT DATA", accessing 510 model data representing a trained model having been trained on training data, metadata associated with the model data and comprising a numerical characteristic, wherein the numerical characteristic is determined based on an intermediate output of the trained model when applied to the training data, and input data to which the trained model is to be applied. The method 500 may further comprise, in an operation titled "APPLYING TRAINED MODEL TO INPUT DATA", applying 520 the trained model to the input data to obtain a further intermediate output of the trained model. The method 500 may further comprise, in an operation titled "DETERMINING CONFORMANCE OF INPUT DATA", determining 530 whether the input data conforms to the numerical characteristic of the training data of the trained model based on the further intermediate output. Such determining of (non)conformance may, for example, involve comparing a probability **P(U_{new}|T)** to a pre-set threshold θ, as explained earlier with Fig. 3 and elsewhere. The method 500 may further comprise, in an operation titled "GENERATING OUTPUT SIGNAL INDICATIVE OF NON-CONFORMANCE", if the input data is determined not to conform to the numerical characteristic, generating 540 an output signal indicative of said non-conformance.

It will be appreciated that, in general, the operations of method 400 of Fig. 4 and/or method 500 of Fig. 5 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in **Fig. 6****,** instructions for the computer, e.g., executable code, may be stored on a computer readable medium 600, e.g., in the form of a series 610 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 6 shows an optical disc 600. Alternatively, the computer readable medium 600 may comprise transitory or non-transitory data 610 representing metadata as described elsewhere in this specification.

### References

Diederik P Kingma, M. W. (2013). Auto-Encoding Variational Bayes. Arxiv, arXiv:1312.6114 .
Goodfellow, I. J. (2014). Generative Adversarial Networks. arXiv, arXiv:1406.2661.
Yu, Y et al (2017). Open-Category Classification by Adversarial Sample Generation. Proceedings of the Twenty-Sixth International Joint Conference on Artificial Intelligence (IJCAI-17)
Dai, Z. et al (2017). Good Semi-supervised Learning That Requires a Bad GAN. 31st Conference on Neural Information Processing Systems (NIPS 2017), Long Beach, CA, USA.
Wang P. et al (2018). Incorporating GAN for Negative Sampling in Knowledge Representation Learning. AAAI Publications, Thirty-Second AAAI Conference on Artificial Intelligence
Zheng, P. et al (2018). One-Class Adversarial Nets for Fraud Detection. arXiv:1803.01798v2 [cs.LG] 5 Jun 2018.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for processing a trained model, comprising:
- a data interface (120) for accessing:
- model data (050) representing a trained model, and
- training data (030) on which the trained model is trained;
- a processor subsystem (140) configured to:
- characterize the training data by:
- applying the trained model to the training data to obtain intermediate output of the trained model, and
- determining a numerical characteristic based on the intermediate output of the trained model;
- encode the numerical characteristic as metadata (060); and
- associate the metadata with the model data to enable an entity applying the trained model to input data to determine whether the input data conforms to the numerical characteristic of the training data of the trained model.

2. The system (100) according to claim 1, wherein the trained model is a trained neural network, and wherein the intermediate output comprises activation values of a select subset of hidden units of the trained neural network.

3. The system (100) according to claim 2, wherein the training data (030) comprises multiple training data objects, and wherein the processor subsystem (140) is configured to:
- apply the trained model to individual ones of the multiple training data objects to obtain multiple sets of activation values; and
- determine the numerical characteristic as a probability distribution of the multiple sets of activation values.

4. The system (100) according to claim 3, wherein the processor subsystem (140) is configured to:
- obtain out-of-spec data comprising multiple out-of-spec data objects which are different from the multiple training data objects;
- apply the trained neural network to individual ones of the multiple out-of-spec data objects to obtain further multiple sets of activation values; and
- select the subset of hidden units to establish a difference, or to increase or maximize the difference, between a) the probability distribution of the multiple sets of activation values and b) a probability distribution of the further multiple sets of activation values.

5. The system (100) according to claim 4, wherein the processor subsystem (140) is configured to select the subset of hidden units by a combinatorial optimization method which optimizes the difference between a) the probability distribution of the multiple sets of activation values and b) the probability distribution of the further multiple sets of activation values, as a function of selected hidden units.

6. The system (100) according to claim 5, wherein the processor subsystem (140) is configured to express the difference as or based on at least one of the group of:
- a Kullback-Leibler divergence measure,
- a cross entropy measure, and
- a mutual information measure.

7. The system (100) according to any one of claims 4 to 6, wherein the processor subsystem (140) is configured to:
- use a generator part of a generative adversarial network to generate negative samples on the basis of the training data;
- generate the out-of-spec data from the negative samples.

8. The system (100) according to any one of claims 1 to 7, wherein the processor subsystem (140) is configured to generate the model data (050) by training a model using the training data (030), thereby obtaining the trained model.

9. The system (100) according to any one of claims 1 to 8, wherein the training data (030) comprises multiple images, and wherein the trained model is configured for image classification or image segmentation.

10. A computer-implemented method (400) of processing a trained model, comprising:
- accessing (410):
- model data representing a trained model, and
- training data on which the trained model is trained;
- characterizing (420) the training data by:
- applying (430) the trained model to the training data to obtain intermediate output of the trained model, and
- determining (440) the numerical characteristic based on the intermediate output of the trained model;
- encoding (450) the numerical characteristic as metadata; and
- associating (460) the metadata with the model data to enable an entity applying the trained model to input data to determine whether the input data conforms to the numerical characteristic of the training data of the trained model.

11. A system (200) for using a trained model, comprising:
- a data interface (220) for accessing:
- model data (050) representing a trained model having been trained on training data,
- metadata (060) associated with the model data and comprising a numerical characteristic, wherein the numerical characteristic is determined based on an intermediate output of the trained model when applied to the training data, and
- input data (040) to which the trained model is to be applied;
- a processor subsystem (240) configured to:
- apply the trained model to the input data to obtain a further intermediate output of the trained model;
- determine whether the input data conforms to the numerical characteristic of the training data of the trained model based on the further intermediate output; and
- if the input data is determined not to conform to the numerical characteristic, generate an output signal (242) indicative of said non-conformance.

12. The system (200) according to claim 11, further comprising an output interface (260) for outputting the output signal (242) to a rendering device (280) for rendering the output signal in a sensory perceptible manner to a user.

13. The system (200) according to claim 11 or 12, wherein the trained model is a trained neural network, wherein the numerical characteristic is a probability distribution obtained from multiple sets of activation values of a subset of hidden units of the trained neural network, wherein the multiple sets of activation values are obtained by applying the trained model to the training data, wherein the further intermediate output of the trained model comprises a further set of activation values of the subset of hidden units, and wherein the processor subsystem (240) is configured to:
- determine a probability of the further set of activation values based on the probability distribution; and
- determine whether the input data conforms to the numerical characteristic of the training data of the trained model as a function of the probability.

14. A computer-implemented method (500) of using a trained model, comprising:
- accessing (510):
- model data representing a trained model having been trained on training data,
- metadata associated with the model data and comprising a numerical characteristic, wherein the numerical characteristic is determined based on an intermediate output of the trained model when applied to the training data, and
- input data to which the trained model is to be applied;
- applying (520) the trained model to the input data to obtain a further intermediate output of the trained model;
- determining (530) whether the input data conforms to the numerical characteristic of the training data of the trained model based on the further intermediate output; and
- if the input data is determined not to conform to the numerical characteristic, generating (540) an output signal indicative of said non-conformance.

15. A computer-readable medium (600) comprising transitory or non-transitory data (610) representing instructions arranged to cause a processor system to perform the computer-implemented method according to claim 10 or 14.
